# EUROPEAN PATENT SPECIFICATION

(11) **EP 1 857 543 B1**
(45) Date of publication and mention of the grant of the patent: **04.04.2012**
(21) Application number: 06713702.6
(22) Date of filing: 14.02.2006
(51) Int. Cl.: C12M 3/04

(54) **Method and apparatus for producing high-density cultured tissue**
Verfahren und Gerät zur Herstellung von kultiviertem Gewebe mit hoher Dichte
Procédé et appareil pour à produire un tissu de culture de densité élevée

(30) Priority: 15.02.2005 JP 2005038261
(43) Date of publication of application: 21.11.2007
(73) Proprietor: School Juridical Person Kitasato Gakuen, Tokyo, 1088641 (JP)
(72) Inventor: ADACHI, Eijiro, Sagamihara-shi, Kanagawa 2288555 (JP); OHASHI, Shihoka, Yokohama-shi, Kanagawa 224-0001 (JP); HIRAI, Kazuya, Sagamihara-shi, Kanagawa 2288555 (JP)
(74) Representative: Horner, Martin Grenville
(86) International application number: PCT/JP2006/302561
(87) International publication number: WO 2006/088029

(56) References cited:
- EP-A- 0 365 313
- GB-A- 2 401 612
- JP-A- 06 277 050
- JP-A- 07 298 876
- JP-A- 10 052 261
- JP-A- 2001 120 255
- JP-A- 2002 142 752
- JP-A- 2002 335 949
- JP-A- 2003 047 461
- JP-A- 2003 265 169
- US-A- 4 720 462
- DATABASE WPI Week 199444 Thomson Scientific, London, GB; AN 1994-353735 XP002595763 & JP 6 277050 A (KANEGAFUCHI KAGAKU KOGYO KK) 4 October 1994 (1994-10-04)

## Description

### TECHNICAL FIELD

The present invention relates to a method of producing a high-density cultured tissue and an apparatus for high-density culturing. More specifically, the present invention relates to a method of producing a high-density cultured tissue for regenerative medicine such as artificial organ, artificial bone and artificial skin or for various experimentations, a high-density cultured tissue obtained by this method and an apparatus for using this production method.

### BACKGROUND ART

In late years, it has come to be possible to culture various cells out of the living body but techniques to sterically and organically dispose these cells are limited to relatively homogeneous tissues such as liver. Conventionally, techniques proposed as three-dimensional culture method only include a method consisting of preparing an adhesive substrate(scaffolding) beforehand, disseminating cells on this substrate and culturing the cells in culture liquid(for example, Japanese Patent Laid-Open No. 06-277050(Patent Document 1), Japanese Patent Laid-Open No. 10-52261(Patent Document 2), Japanese Patent Laid-Open No. 2001-120255(Patent Document 3), Japanese Patent Laid-Open No. 2003-265169(Patent Document 4), W02004-078954(Patent Document 5), Japanese Patent Laid-Open No. 2004-65087(Patent Document 6)), or a method of mixing and culturing cells and an adhesive substrate on a dish (Petri dish).

In the former case, however, it is necessary to have the cells migrate into the adhesive substrate and to continue culturing for a long term. In the latter case, the adhesive substrate is a very sparse tissue and culturing should be continued for a long term until the disseminated cells constrict the substrate and attain a highly dense state. With either method adopted, culturing period of around two weeks is necessary, and cells secret enzymes which decompose the adhesive substrate during the term, which may lead to decomposition of once formed high-density tissues.
As described above, although three dimensionally highly densified cultured tissues are expected to be useful in medical transplantation, experimentations of bioscience, clinical trial of a new medicine, etc., they are not sufficiently put to practical use up to now for the reasons that they require a long term to prepare but allow a short time to use.

Patent document 1: Japanese Patent Laid-Open No. 06-277050
Patent document 2: Japanese Patent Laid-Open No. 10-52261
Patent document 3: Japanese Patent Laid-Open No. 2001-120255
Patent document 4: Japanese Patent Laid-Open No. 2003-265169
Patent document 5: WO2004-078954
Patent document 6: Japanese Patent Laid-Open No. 2004-65087
Moreover the documents GB-A-2401612 and US 4720462 a disclose culture systems for growing animal cells in cylindrical or horizontal mesh devices.

### DISCLOSURE OF THE INVENTION

### (Problems to be Solved by the Invention)

Therefore, an object of the present invention is to provide a method of rapidly preparing a body-tissue-like artificial tissue comprising highly densely accumulated cells by a simple operation. In particular, the present invention aims at enabling to prepare various tissues for transplantation useful as materials for regenerative medicine as well as tissues which can replace and/or supplement animal experiments performed during the development of cosmetics and new drugs readily and rapidly in a medical scene.

### (Means for solving the problems)

The present inventors have conducted intensive studies for solving the above-mentioned problems, consequently found that the formation of the substrate to which cells adhere and the dissemination of the cells can be performed at a time by disposing a mesh member and a liquid flow controlling member within a path in which a cell culture liquid containing an extracellular matrix component and one or more kinds of animal cells is subjected to circulation culturing so that the liquid flow controlling member is disposed in contact with or close to the mesh member on the back side thereof against the direction of the liquid flow and that the desired tissue can be prepared markedly more rapidly as compared with the conventional methods by applying the above constitution, and thus completed the present invention.

That is, the present invention provides the following methods of producing a high-density cultured tissue, high-density cultured tissues obtained by these methods and apparatuses to be used for these methods.
1. A method of producing a high-density cultured tissue characterized in that the method comprises disposing a mesh member and a liquid flow controlling member within a path in which a cell culture liquid containing an extracellular matrix component and one or more kinds of animal cells is subjected to circulation culturing so that the liquid flow controlling member is disposed in contact with or close to the mesh member on the back side thereof with regard to the direction of the liquid flow, thereby accumulating the polymerized extracellular matrices and the animal cells highly densely on the surface of the mesh member.
2. The method of producing a high-density cultured tissue according to above 1 wherein the extracellular matrix component contains one of collagen, elastin, proteoglycan, fibrillin, fibronectin, laminin, chitin, chitosan and chemically modified compounds thereof or a mixture of two or more of these compounds.
3. The method of producing a high-density cultured tissue according to above 1 wherein the animal cell is one of somatic cells, tumor cells and embryonic stem cells or a mixture of two or more of these cells.
4. The method of producing a high-density cultured tissue according to above 3 wherein the somatic cell is selected from fibroblast, liver cell, blood vessel endothelial cell, epidermic cell, chondrocyte, neuroglia and smooth muscle cells.
5. The method of producing a high-density cultured tissue according to above 1 wherein the culture liquid further comprises a biologically active substance.
6. The method of producing a high-density cultured tissue according to above 5 wherein the biologically active substance is one of cell growth factors, hormones and/or natural or synthetic chemical substances having a pharmacological effect or a mixture containing two or more of them.
7. The method of producing a high-density cultured tissue according to above 1 wherein the culture temperature is 35 to 40°C and the culture time is 6 hours to 9 days.
8. The method of producing a high-density cultured tissue according to above 1 wherein the liquid flow controlling member is a porous material through which the liquid flow can penetrate.
9. The method of producing a high-density cultured tissue according to above 7 wherein the porous material through which the liquid flow can penetrate is a filter-paper, a nonwoven fabric or a silk fibroin film.
10. The method of producing a high-density cultured tissue according to above 1 wherein the mesh member is made of a metal, a ceramic, a synthetic resin or a natural material.
11. The method of producing a high-density cultured tissue according to above 1 wherein the liquid flow controlling member is incorporated with the mesh member.
12. The method of producing a high-density cultured tissue according to above 11 wherein the liquid flow controlling member incorporated with the mesh member is an artificial blood vessel.
13. The method of producing a high-density cultured tissue according to above 1 wherein the mesh member and the liquid flow controlling member are cylindrical and the mesh member is disposed inside of the liquid flow controlling member in the liquid flow from the cylindrical center toward the outer circumference.
14. The method of producing a high-density cultured tissue according to above 13 wherein the mesh member and the liquid flow controlling member are cylindrical and coaxially disposed, the culture liquid is run from the central axis thereof toward the outer circumference and the culture liquid collected at the outer circumference is circulated to the central axis, thereby accumulating the extracellular matrix molecules and the animal cells highly densely on the surface of the mesh member.
15. The method of producing a high-density cultured tissue according to above 1 wherein the mesh member and the liquid flow controlling member are planar and parallelly disposed and the liquid flow controlling member is disposed in contact with or close to the mesh member on the back side thereof with regard to the direction of the liquid flow.
16. The method of producing a high-density cultured tissue according to above 15 wherein the mesh member and the liquid flow controlling member are horizontally disposed with one above the other in a downward liquid flow.
17. A method of producing a high-density cultured tissue comprising producing a high-density cultured tissue by a method described in any of above 1 to 16, taking out the obtained high-density cultured tissue and continuing culturing in a non-circulated culture liquid which contains an extracellular matrix component and one or more kinds of animal cells in the same or different formulation.
18. A method of producing a high-density cultured tissue comprising producing a high-density cultured tissue by a method described in any of above 1 to 16, taking out the obtained high-density cultured tissue and performing at least one operation of forming a different high-density cultured tissue on the above tissue using the same or different culture liquid which contains an extracellular matrix component and one or more kinds of animal cells, thereby forming a laminate type high-density cultured tissue.
19. A method of producing a laminate type high-density cultured tissue comprising taking out a laminate type high-density cultured tissue produced by a method described in above 18 and continuing culturing in a non-circulated culture liquid which contains an extracellular matrix component and one or more kinds of animal cells in the same or different formulation.
20. The method of producing a high-density cultured tissue according to any of above 1 to 19 wherein the high-density cultured tissue is skin, cartilage, blood vessel, nerve, muscle or various internal organs.
21. An apparatus for high-density cell culturing which comprises a liquid tank to accommodate a cell culture liquid containing an extracellular matrix component and one or more kinds of animal cells; pump means to circulate the cell culture liquid; a reactor having a cylindrical vessel as a main body and conduit lines which link these elements to constitute a closed circuit, wherein the reactor coaxially keeps a cylindrical mesh member and a cylindrical liquid flow controlling member inside thereof so that the mesh member may be positioned inside the liquid flow controlling member and they may be in contact with or close to each other, and the reactor has an inlet and an outlet of the liquid for circulating the cell culture liquid through the mesh member from the inside to the outside to accumulate the polymerized extracellular matrx molecules and the animal cells on the mesh member.
22. An apparatus for high-density cell culturing which comprises a liquid tank to accommodate a cell culture liquid containing an extracellular matrix component and one or more kinds of animal cells; pump means to circulate the cell culture liquid; a reactor having a cylindrical vessel as a main body and conduit lines which link these elements to constitute a closed circuit, wherein the reactor horizontally keeps a planar mesh member and a planar liquid flow controlling member inside thereof with one above the other so that the mesh member and the liquid flow controlling member may be in contact with or close to each other, and the reactor has an inlet and an outlet of the liquid for circulating the cell culture liquid through the mesh member from the above to the bottom to accumulate the extracellular matrix molecules and the animal cells on the mesh member.

### (Effects of the Invention)

According to the present invention, body-tissue-like artificial tissues in which extracellular matrix and animal cells are highly densely accumulated can be prepared rapidly by a simple operation. The present invention enables to prepare various tissues for transplantation useful as materials for regenerative medicine as well as tissues which can replace and/or supplement animal experiments performed during the development of cosmetics and new drugs readily and rapidly in a medical or research scene.

### BRIEF DESCRIPTION OF THE DRAWINGS

Fig. 1 is a schematic view showing an example of constituting a reactor part of the culture apparatus to be used in the method of the present invention.
Fig. 2 is a schematic view showing another example of constituting a reactor part of the culture apparatus to be used in the method of the present invention.
Fig. 3 is a schematic view showing another example of constituting a reactor part of the culture apparatus to be used in the method of the present invention.
Fig. 4 is a schematic view showing an example of constituting a culture apparatus to be used in the method of the present invention.
Fig. 5 is a photograph just as visually observed of a high-density cultured tissue (Example 1) obtained by the method of the present invention.
Fig. 6 is an electron microgram of a high-density cultured tissue (Example 1) obtained by the method of the present invention.
Fig. 7 is an optical microgram of a high-density cultured tissue (Example 2) obtained by the method of the present invention.
Fig. 8 is a photograph of a high-density cultured tissue obtained by the method of the present invention inside of the reactor.
Fig. 9 is an optical microgram of a high-density cultured tissue (Example 3) obtained by the method of the present invention.
Fig. 10 is a photograph just as visually observed of a high-density cultured tissue (Example 4) obtained by the method of the present invention.
Fig. 11 is a photograph just as visually observed of a high-density cultured tissue (Example 5) obtained by the method of the present invention (wherein the arrow indicates a fibroblast and * indicates a smooth muscle cell; hematoxylin-eosin staining).
Fig. 12 is an optical microgram of a laminated high-density cultured tissue (Example 5) obtained by the method of the present invention.

### BEST MODE FOR CARRYING OUT THE INVENTION

In the present invention, a mesh member and a liquid flow controlling member are disposed within a path in which a cell culture liquid containing an extracellular matrix component and one or more kinds of animal cells is subjected to circulation culturing so that the mesh member and the liquid flow controlling member is disposed in contact with or close to each other. In this construction, the mesh member is disposed upstream with regard to the flow of the culture liquid and thereby accumulating the extracellular matrix molecules and the animal cells highly densely on the surface of the mesh member.

The flow rate of the culture liquid is locally decreased by disposing a mesh member and a liquid flow controlling member so that they may be in contact with or close to each other. Consequently, concentrations of the extracellular matrix component and animal cells suspended in the cell culture liquid are locally increased, and as a result, the extracellular matrix component and animal cells are highly densely accumulated on the mesh member.

In order to achieve the high-density accumulation of the extracellular matrix component and animal cells homogeneously, the culture liquid flow should be run generally homogeneously for the mesh member and the liquid flow controlling member. This can be realized, as the first Example, by designing the mesh member and the liquid flow controlling member as cylindrical members, coaxially disposing them with the mesh member inside the liquid flow controlling member and running the culture liquid from the inside of the mesh member to the outside thereof. It can also be realized, as the second Example, by designing the mesh member and the liquid flow controlling member as planar members, parallelly disposing them and running the culture liquid generally orthogonally to the surface of the mesh member.

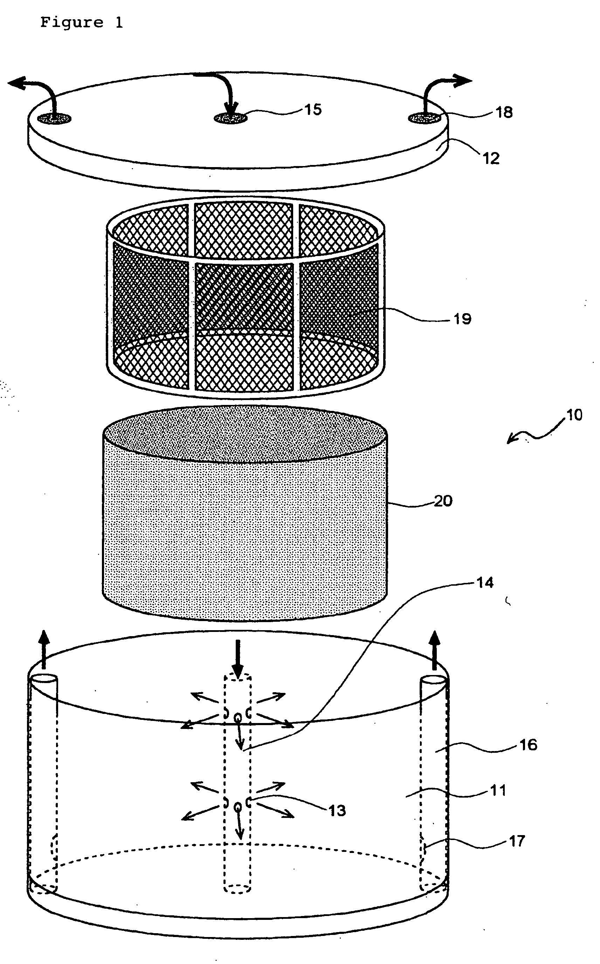

The first Example is preferably an Example in which the culture liquid flow is radially run from the central axis thereof toward the mesh member and the liquid flow controlling member disposed in the cylindrical form in particular. Such an Example can be typically realized with a cylindrical reactor 10 shown in Fig. 1. This reactor basically comprises a main body of cylindrical vessel 11 and a lid 12, and the main body of vessel is provided with a center pipe 14 having plural holes 13 on the surface thereof whereas the lid 12 is provided with an opening 15 in the center thereof, and when the reactor is tightly sealed with the lid 12, the opening 15 connects with the hollow part of the center pipe 14 to form a flow channel of the liquid. Alternatively, the central pipe 14 may be incorporatedly fixed to the lid 12 along with the opening 15. In addition, another conduit line 16 connecting the inside of the vessel with the outside is provided in the outer circumference of the main body of the vessel 11. One or a plural number of this conduit line 16 may be provided, each has a bored hole 17, and the upper end of the conduit line 16 is connected with an opening 18 bored at the corresponding position on the lid 12 to form a channel of the liquid. This enables to introduce liquid from the opening 15 into the reactor 10 and to take out the liquid from the opening 18. However, any configuration for taking out the liquid from the outer circumference may be adopted, and it is not limited to the constitution described here.

A cylindrical mesh member 19 is disposed approximately coaxially around the above central pipe 14, and a liquid flow controlling member 20 is disposed between the outer surface of the above cylindrical mesh member and the inner surface of the vessel in the present invention. Fig. 1 particularly shows the mesh member 19 and the liquid flow controlling member 20 but the cylindrical mesh member and the liquid flow controlling member are mounted in the vessel with the former being positioned inside the latter when they are used. For example, as illustrated in the drawing, the cylindrical mesh member 19 can be formed by fixing or attaching a mesh to the inside of a frame, and the liquid flow controlling member which is a flexible sheet or a film can be wound around the outside of the frame for the mesh member 19.
The cell culture liquid containing an extracellular matrix component and animal cells comes out from the surface holes 13 into the internal space of the vessel in the radial direction by sending the culture liquid to the reactor 10 from the above opening 15 (shown by the arrow in the drawing). The culture liquid passes through the mesh member 19 and the liquid flow controlling member 20, and it is collected at the inner surface of the vessel and circulated to the above center pipe through pump means as described later. Preferably, a device sensing the pressure in the circuit is provided in the circulation path.

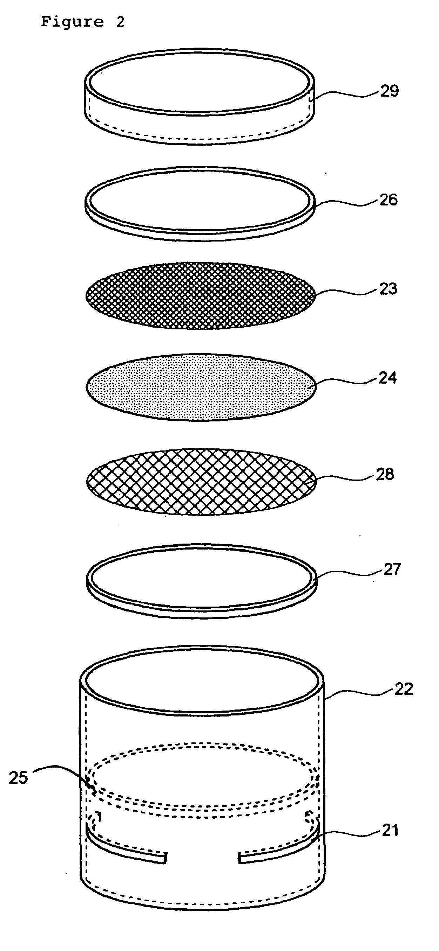

As the second embodiment, particularly preferred is an embodiment in which the culture liquid flow is run to the mesh member and liquid flow controlling member which are planar members disposed in parallel from the mesh member side. Such an embodiment is realized, for example, by installing a cylindrical member 22 having plural openings 21 in the lower part thereof in a flow path as shown in Fig. 2.
In the present invention, a planer member 23 composed of a mesh and a liquid flow controlling member 24 below the planer member 23 are disposed in a cylindrical member 22. Preferably, the cylindrical member 22 has a rib 25 on the inner circumference thereof, and members 26, 27 (for example, silicone rings) for preventing leakage and a supporting mesh 28 are placed, as required, and a liquid flow controlling member 26 and a planar mesh member 24 are placed on the top. Another member (for example, inner cylinder 29) for preventing leakage of the liquid is further placed on it. Fig. 2 particularly shows these members but when they are used, theses members are mounted on the rib 25 in the cylindrical member 22 so that they may be fixed and installed in the flow path as described above.
For example, the central pipe 14 is removed from the vessel of the reactor 10 of the first embodiment and the above cylindrical member 22 is installed in the vessel. When the liquid entering the reactor flows outside of the cylindrical member 22, efficiency in the formation of high-density tissue decreases. Therefore, it is preferable to design so that the lid of the reactor and the cylindrical member 22 or the inner cylinder 29 may be in close contact by adjusting the height of the cylindrical member 22 or the inner cylinder 29. The liquid entering the cylindrical member 22 flows out of plural openings 21 positioned at the lower part of the reactor vessel and the liquid is collected at the inner surface of the vessel in the same way as in the first embodiment. After a high-density tissue has been formed, the cylindrical member 22 is taken out of the reactor vessel and pushed up with a protruded member whereby the mesh member 24 can be easily taken out.

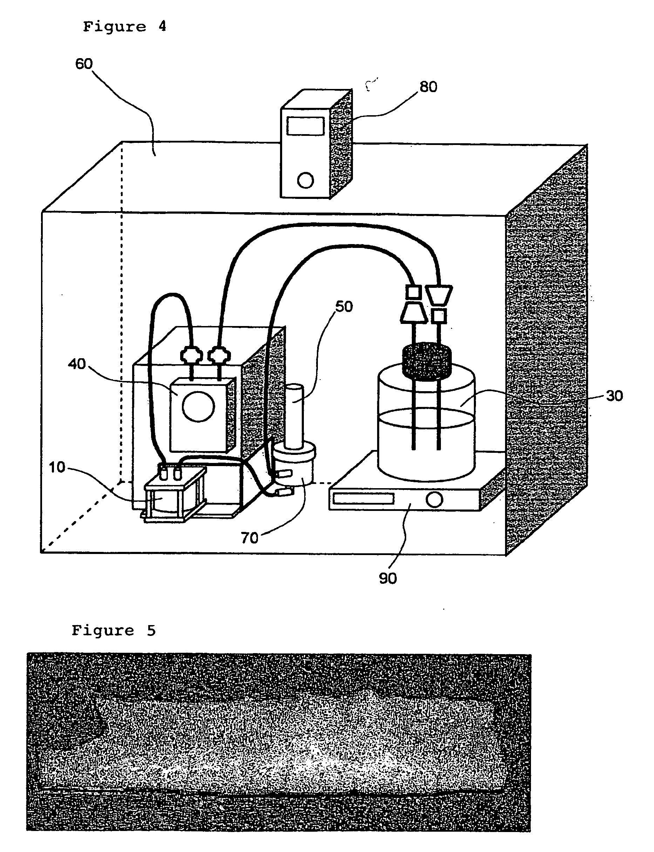

An example of the constitution of the whole apparatus include a closed circuit type culture apparatus comprising a reactor 10, a culture medium tank 30, a circulation pump 40, a flow cell 50 all of which are connected with conduit lines and installed in a incubator 60 as shown in Fig. 4. Preferably, sensors such as a DO (dissolved oxygen) sensor 70, a display unit 80 for displaying the measured values, and further a stirrer 90 for stirring the culture medium in the culture medium tank 30 are installed.
The stirrer 90 is, for example, a magnetic rotating device turning a magnetic stirring bar put in the culture medium tank.

The reactor vessel exemplified above is described in Japanese Examined Patent Publication No. H02-109966, and there exist commercially available products, but they are used by filling up living body supported catalysts in a vessel and the culture medium is run from the outer circumference to the inside, and it is not intended to be used as a high-density cell culture apparatus following the constitution prescribed by the present application. According to the studies by the present inventors, an unexpected finding that high-density cell culture can be realized in a shorter time than the conventional methods particularly by the constitution to run the culture medium from the inside to the outer circumference.

The mesh member can be anything which can sufficiently support the high-density cell culture which is a mixture of an extracellular matrix component and animal cells, and it is usually a member having a mesh which does not significantly block the liquid flow. Specifically, the mesh has holes of around 100 µm to 1 mm, more preferably around 100 µm to 0.5 mm. For example, meshes of around 100 µm to 300 µm formed by weaving a wire having a diameter of around 0.08 to 0.1 mm can be used. The material of the mesh member may be any of metals (for example, stainless steel), synthetic resins (for example, polyester), ceramics and the other artificial materials. A metal mesh, which is readily sterilized and cleaned, is usually preferable, but, for example, when a biocompatible material such as an artificial blood vessel material is used, high-density cellular tissue can be formed on it and applied to the living body.

The liquid flow controlling member is not particularly limited as long as it is a member which can pass and slow down a liquid flow but usually it is a porous material through which the liquid flow can penetrate, particularly a porous film through which the liquid flow can penetrate. Examples of such a film include a filter-paper, a fabric cloth, a nonwoven fabrics and a silk fibroin film.

The mesh member and the liquid flow controlling member are disposed in contact with or in close to (in proximity to) each other in the present invention. The proximity as used herein refers to a distance at which the liquid flow controlling member can cause stagnation of the solution and it is usually around several millimeters or less, preferably about 1 mm or less. Either of the mesh member or the liquid flow controlling member may be disposed upstream (in view of the liquid flow), but when the mesh member (particularly metal mesh) is disposed upstream, a high-density cell culture tissue consisting of an extracellular matrix component and animal cells alone can be obtained easily. In the case that a composite member comprising a high-density cell culture tissue consisting of an extracellular matrix component and animal cells and the liquid flow controlling member is aimed at, the liquid flow controlling member may be disposed upstream. Besides, the mesh member and the liquid flow controlling member may be unified. For example, a conventional artificial blood vessel material has a structure comprising a polyester knit lining a cylinder made of stainless steel, and can be used as a substitute member of the mesh member - liquid flow controlling member of the present invention.

The dimension conditions (area, or diameter in a radial flow type reactor) of the mesh member and the liquid flow controlling member other than the above depend on the kind of the cell or the size of the tissue to bring up and, for example, the conditions to achieve an circulation rate of the cell culture liquid of around 4 to 10 µl/cm²/second, preferably around 6 to 8 µl/cm²/second in the vicinity of the mesh member or the liquid flow controlling member will be satisfactory.

In the present invention, the extracellular matrix component to be contained in the cell culture liquid may be any molecules which can be polymerized or mutually adhered as a substrate for cell adherence at 37°C in a neutral pH area but typically it is a substance present in connective tissues. Examples of such a substance include collagen, elastin, proteoglycan, fibrillin, fibronectin, laminin, chitin and chitosan. These extracellular matrix components may be used singly or as a combination of two or more. Each of the above components can be used after subjected to various kinds of chemical modification. The modification may be modifications usually observed in a living body or artificial modifications to provide various kinds of activity and characteristics. Furthermore, constituents of each of the above components (for example, glycosaminoglycans such as hyaluronic acid, chondroitin sulfate, dermatan sulfate, heparan sulfate, heparin, keratan sulfate for proteoglycan,) can be also included.

Preferred are collagen or elastin or combinations of these with one or more kinds of the above components, and particularly preferred are collagen or combinations of collagen with one or more kinds of the above components. Preferred component can be decided depending on the type of the target cultured tissue.

Any type of conventionally known collagen can be used as a collagen. For example, collagens of Type I, Type II, Type III, Type IV, Type V, etc. can be used.
These collagens can be used by using body tissue containing the collagen to be obtained as raw materials and solubilizing them with an acid, an enzyme, an alkali and the like. It is also preferable to remove the whole or part of telopeptide at the molecular end by enzymatic treatment for eliminating or suppressing allergic reaction and/or rejection. Examples of such collagen materials include Type I collagen derived from pig skin, Type I collagen derived from pig tendon, type II collagen derived from bovine nasal cartilage, Type I collagen extracted from fish, genetically modified collagen and mixtures of these. These are, however, examples for illustration and the other kinds of collagens can be also used depending on the purpose. For example, Type IV collagen is used when a tissue corresponding to basement membrane is formed.

The animal cells to be contained in the cell culture liquid are not limited in particular and appropriately selected depending on the purpose, and examples thereof include somatic cells, neoplastic cells, and embryonic stem cells. Examples of the somatic cells include fibroblasts, liver cells, blood vessel endothelial cells, epidermic cells, epithelial cells, chondrocytes, neuroglial cells and smooth muscle cells. These can be used singly or a mixture of two or more of these can be used.

The basic composition of the cell culture liquid depends on the kind of the target animal cell to culture, and conventional natural media or synthetic culture media can be used. Synthetic culture media are preferable in consideration of the factors such as bacterial or viral infection from animal origin materials and variation of the composition caused by the difference of supply time and place. The synthetic culture media are not limited in particular, but examples thereof include α-MEM (Minimum Essential Medium), Eagle MEM, Dulbecco MEM (DMEM), RPMI1640 culture medium, CMRC culture medium, HAM culture medium, DME/F12 culture medium, 199 culture mediums and MCDB culture medium. Serum and the other conventionally used materials may be appropriately added. As natural media, any of conventional natural media can be usually used and they are not limited in particular. One of these can be used alone or a mixture of two or more of these can be used in combination.

The content of the extracellular matrix component in the cell culture liquid is 0.1 to 0.5 mg/ml at the time of starting culture and preferably around 0.2 to 0.3 mg/ml.

The cell culture liquid may also contain other materials promoting cell adhesion, in addition to the above extracellular matrix component, for example, peptides and proteins such as polylysine, histone, gluten, gelatine, fibrin, fibroin; cell adhesive oligopeptides such as RGD, RGDS, GRGDS, YIGSR, IKVAV or synthetic proteins in which these sequences incorporated by genetic engineering; polysaccharides such as alginic acid, starch, dexetrine and derivatives of these; polymers of lactic acid, glycolic acid, caprolactone and hydroxybutyrate or copolymers thereof and biodegradable polymers such as block copolymers of these polymers and copolymers with polyethylene glycol or polypropylene glycol.

The culture liquid may also contain biologically active substances other than the above. Examples of such a biologically active substance include cell growth factors, hormones and/or natural or synthetic chemical substances having pharmacologic effects. Functions can be added or varied by adding such materials. In addition, cell incorporated in tissues containing a synthetic compound which does not occur in nature can be formed by changing circulation conditions.

The cell growth factor is not particularly limited, and examples thereof include epidermal growth factor, epidermal growth factor, fibroblast growth factors, platelet-derived growth factor, hepatocyte growth factor and insulin. The other cell growth factors can be used depending on the kind of the cell to be cultured.

The hormone is not limit it in particular but examples thereof include insulin, transferrin, dexamethasone, hydrocortisone, thyroxine, 3,3',5-tri-iodothyronine, 1-methyl-3-butylxanthine and progesterone. One of these can be used alone or a mixture of two or more of these can be used in combination.

Examples of the other biologically active substances include ascorbic acid (in particular, L-ascorbic acid), biotin, calcium pantothenate, ascorbic acid-2-phosphate, vitamin groups such as vitamin D, serum albumin, proteins such as transferrin, lipids, fatty acid sources, linoleic acid, cholesterol, pyruvic acid, nucleosides for synthesizing DNA and RNA, glucocorticoid, retinoic acid, β-glycerophosphate, monothioglycerol and various antibiotics. These are, however, examples for illustration and the other kinds of components can be also used depending on the purpose. One of the above components can be used alone or a mixture of two or more of these can be used in combination.

Culturing can be performed till a high-density cultured tissue of a desired size (thickness) is obtained by an ordinary condition. Typically, culture temperature is 35 to 40°C and culture time is 9 hours to 6 days. As described above, conventional production methods of high-density cultured tissue require periods of more than two weeks. According to the present invention, necessary culture time is largely shortened.

In addition, the present invention also provides a method of producing a high-density cultured tissue comprising producing a high-density cultured tissue by a method described in any of the above, taking out the obtained high-density cultured tissue and continuing culturing in a non-circulated culture liquid which contains an extracellular matrix component and one or more kinds of animal cells in the same or different formulation. Here, a non-circulation culture condition is, for example, culture on a dish. By adopting such a method, newly stacked cells are expected to proliferate in a similar condition as in the living body.

The present invention also provides a method of producing a high-density cultured tissue comprising producing a high-density cultured tissue by a method described in any of the above, taking out the obtained high-density cultured tissue or not taking out the obtained high-density cultured tissue and performing at least one operation of forming a different high-density cultured tissue on the above tissue using the same or different culture liquid which contains an extracellular matrix component and one or more kinds of animal cells, thereby forming a laminate type high-density cultured tissue.

For example, culturing can be performed by changing the kind and/or the concentration of the extracellular matrix component, the kind and/or the concentration of the nourishing ingredient or the kind and/or the concentration of the ingredients added or culture conditions such as temperature and pH continuously or uncontinuously, thereby forming an extracellular matrix environment which is more close to that in the living body in a culture apparatus. In addition, tissues having a certain graded structure such as bowel, ureter or blood vessels can be regenerated by casting plural types of cells (for example, smooth muscle cells and blood vessel endothelial cells) as well as cell adhesion matrix into a closed circuit type culture apparatus at the same time or with a time lag.

Furthermore, the laminate type high-density cultured tissue produced by this method is taken out and culturing can be continued in a non-circulated culture liquid which contains an extracellular matrix component and one or more kinds of animal cells in the same or different formulation.

In this way, the present invention enables to form a homogeneous high-density cultured tissue rapidly and reliably as well as to form a high-density cultured tissue incorporating or compositing plural structures rapidly and reliably. Such high-density cultured tissues include tissues of each part of human body, and, for example, skin, cartilage, blood vessel, nerve, ureter, heart, skeletal muscle and various internal organs and tumor tissues.

### Examples

Hereinbelow the present invention is more specifically described by way of Examples.

### Example 1

### [High-density culture apparatus 1]

High-density culture apparatus 1 was a type as shown in Fig. 1 and constituted as follows.

A piece of filter-paper (JIS P-3801 #1 manufacture by Toyo Roshi Kaisha, Ltd.) was wound around the outer circumference of a cylinder which contains a stainless steel mesh (mesh size: 133×266 µm) having a diameter of 22 mm and a height of 17mm to form a mesh - liquid flow controlling member. The mesh was put on the inside of the support frame, and the mesh and the liquid flow controlling member were separated by around the thickness of the above supporting member at the largest.

This mesh - liquid flow controlling member was inserted in a radial flow type bioreactor (BRK-05 manufacture by Able Co., Ltd.) and a closed circulatory system was constituted along with a culture medium tank, a flow cell, a DO sensor (dissolved oxygen meter), a device for sensing the pressure in the circuit and a circulating pump. The above radial flow type bioreactor comprised a polycarbonate vessel having a volume of about 5 ml, a hollow central axis having plural holes on the surface and an outer circumference surface having plural holes. The vessel is equipped with a lid having a hole connected to the inside of the central axis and a liquid is sent in through this hole.

According to the manual by the manufacturer, this was a device which should be used by filling the inside of the vessel with carriers such as beads or a sponge and sending a culture medium from the outer circumference surface to the central axis. On the contrary in the following experiment, nothing but a mesh - liquid flow controlling member is placed in the vessel, and the apparatus was configured so that the liquid flow may run from the central axis to the outer circumference. These are installed in a CO₂-incubator as shown in Fig. 4 and a culture medium was circulated while the culture medium in the culture medium tank was stirred with a magnetic stirring bar to supply oxygen and preventing gelation.

### [Culture experiment]

Type I collagen (product of Koken Co.,Ltd.; extracted from bovine hide with protease pepsin) was added to 250 ml of a mixture of culture liquid (DMEM+10% FBS (fetal bovine serum) +100 unit/ml penicillin G, 100 µg/ml streptomycin) and mouse normal fibroblast (5.0×10⁷ cells) at a concentration of 0.5 mg/ml. After the collagen was added, the culture liquid was circulated in the above closed circulatory system at a flow rate of 7 ml/min. Five (5) ml of the circulated liquid was collected in every 24 hours and the concentration of Type I collagen and matrix metalloprotease (MMP) activity were respectively analyzed by SDS-PAGE and zymography.

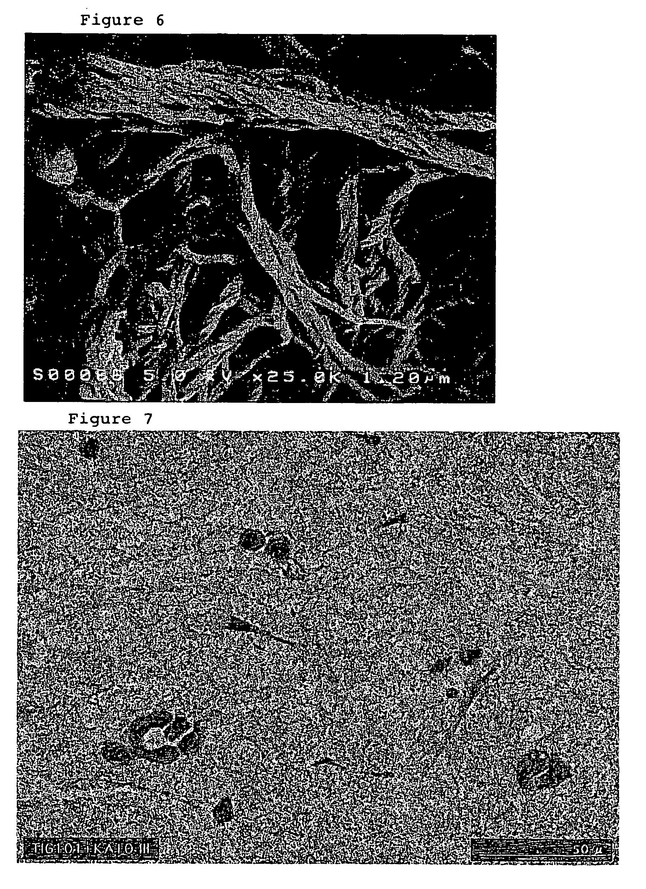

After the circulation was started, a white and smooth material began to accumulate on the mesh side of the above mesh - liquid flow controlling member. The above mesh - liquid flow controlling member was collected from the reactor on the third day, the sixth day and the ninth day after the start of the circulation, an accumulated matter (2 mm in thickness, 17 mm in width and 59 mm in length, about 1.0 g in wet weight) was formed on the mesh as of on the third day (Fig. 5).
After that, some increase in weight was observed but change in the form was not recognized.
Observation of the accumulated matter with an optical microscope (400 times amplification) revealed that the matter consisted of a substrate and cells. The substrate, when observed with a scanning electron microscope (Fig. 6), showed a network structure in which fibrils with a diameter of about 140 nm repeated branching and rejoining. This fiber had periodic striation characteristic to collagen fibrils, and Type I collagen in the culture liquid determined by SDS-PAGE analysis showed decrease with time, and therefore, it was confirmed that the substrate was a self polymer of Type I collagen.

The observed values by the DO sensor showed a tendency to decrease with time after the start of circulation, supporting survival/growth of the cells. By observation under an optical microscope, part of the fibroblasts showed spindle shape from the time point of the third day which morphologically resembled with fibroblasts occurring in the dermal layer. According to the results of hematoxylin-eosin staining and electron microscope observation, the cell concentration in the accumulated matter was around 55 to 70 cells in the visual field of 400 times amplification regardless of the culture days within 3 to 9 days. Transient elevation of MMP-2 and MMP-9 was observed in the analysis by gelatine zymography and cells were observed not in masses in each gel but existed in a diffused state during the nine-day period of culturing.
As described above, the method of the present invention enables to achieve culturing of fibroblast cells in high density in more similar form in the dermis unlike in the culturing on a dish.

### Example 2

250 ml of a culture liquid adjusted to contain 0.5 mg/ml of Type I collagen just same as in Example 1 was prepared and 1.0×10⁷ cell/250ml of human stomach cancer cell (KATO III) and the same number of human fibroblast (TIG101) were circulated in the above closed circuit type culture apparatus for four days and an artificial tissue (1 to 2 mm in thickness, 17mm in width, 59mm in length) was obtained in the same way as in Example 1. Type I collagen dissolved in the culture liquid decreased as circulation culture proceeded as in Example 1, and the results of electron microscopic observation also confirmed that the substrate of the artificial tissue was formed of a self polymer of the Type I collagen.

It was observed with an optical microscope (Fig. 7) that a number of scirrhous cancer cells and fibroblast cells were formed in the network structure of collagen fibrils in a very similar condition as in the human cancer tissue.
In this example, when circulation culture was performed only with scirrhous cancer cells, many of the cancer cells withered and no cancer tissue was obtained. The human cancer tissue model can be created only by performing culture along with normal fibroblast cells in a Type I collagen gel.

### Example 3

In place of the above mesh - liquid flow controlling member of Example 1, an artificial blood vessel plainly woven with collagen-coated Dacron (Intergard-W (trademark)) having approximately the same shape and the same size as the above member was placed in a reactor and culture was performed for five days. The Dacron artificial blood vessel was a product already provided for transplant operation and composed of a knit structure of polyester fiber.

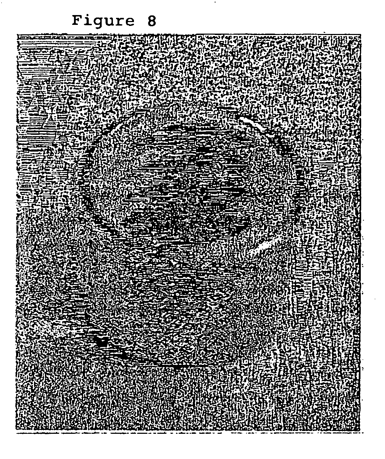

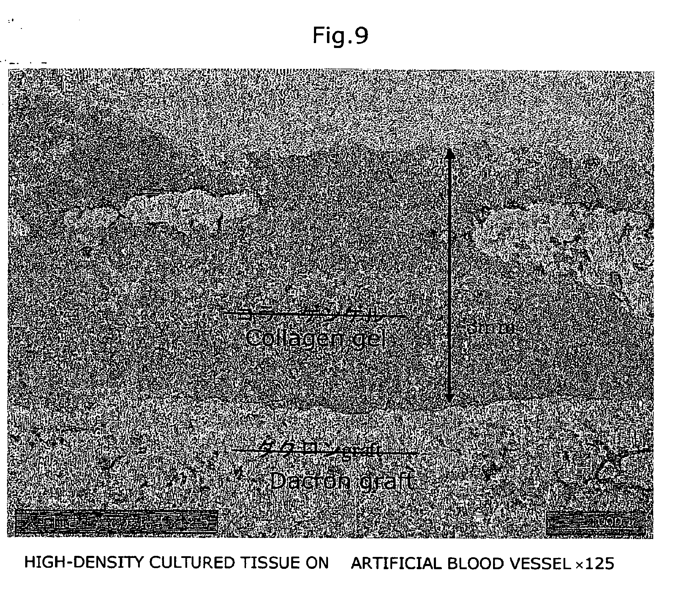

The collagen gel of the collected artificial tissue had a thickness of about 3 mm and homogeneous, and besides it was thicker in comparison with those in Examples 1 and 2 (Fig. 8). Circulated cells were observed in the tissue which had been formed on the Dacron artificial blood vessel when searched for with an optical microscope (Fig. 9).
As described above, the method of the present invention enables to readily form an artificial tissue on an artificial blood vessel, and it also was confirmed that an artificial tissue can be formed in combination with an artificial biopolymer material. In addition, the combination can be replaced with, for example, smooth muscle and Type IV collagen thereby to form a media of a blood vessel (which is a smooth muscle layer) artificially.

### Example 4

### [High-density culture apparatus 2]

High-density culture apparatus 2 was a type as shown in Fig. 2 and constituted as follows.
A metal cylinder of 21.3 mm in outer diameter, 19.3 mm in inner diameter and 4.2 mm in height having three opening of 11.2mm in length and 20.5 mm in height in the lower part thereof was prepared. This metal cylinder was provided with a rib of 1.1mm in width on the inner circumference. A rubber ring (19.3 mm in outer diameter, 17.0 mm in inner diameter, 0.5mm in height) made of silicone, a stainless steel wire netting disc (18.7 mm in diameter (same below), 35 to 50 mesh) for a filter-paper support, a piece of filter paper (JISP-3801 #1 manufacture by Toyo Roshi Kaisha, Ltd.), a stainless steel wire netting disc (100 mesh) for producing a high-density cultured tissue and a rubber ring (19.3 mm in outer diameter, 17.0 mm in inner diameter, 0.7 mm in thickness) made of silicone were stacked in this cylinder, and a metal inner cylinder (19.2 mm in outside diameter, 17.0 mm in inner diameter, 0.5 mm in height) was further disposed inside of the metal cylinder so that the both were contacted. The metal cylinder disposed on the inner side (inner cylinder) was designed to be in close contact with the lower surface of a lid when the reactor was closed with the lid so that the circulated culture liquid entirely might run within the inside of the metal cylinder when the culture liquid was introduced from the opening bored at the center of the lid.

### [Culture experiment]

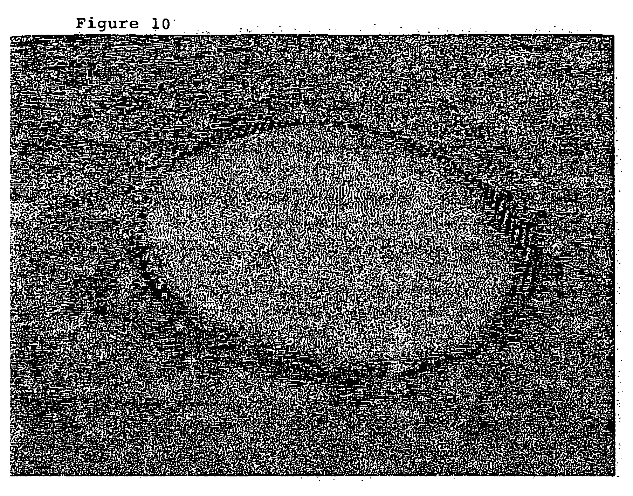

Circulation was performed by introducing the liquid from the center of the reactor lid as above for 20 hours except that the high-density culture apparatus was changed to the above apparatus; the cultured cell to human derived cancer cell (1.0×10⁷ cells); collagen concentration to 0.5 mg/ml; and the total volume of the culture liquid to 200 ml. The culture liquid and the collagen used were the same as those in Example 1. As a result, a white and smooth accumulated matter was obtained on the almost entire surface of the upper mesh (Fig. 10).
After the circulation was stopped, the metal cylinder was removed from the reactor and the cylinder was gently placed on a member composed of a disc base (diameter: 50 mm) equipped with a column metal bar (diameter: 16.5 mm; height: about 25 mm) at the center thereof and the mesh inside of the cylinder was taken out. The accumulated matter on the mesh had a uniform thickness of about 0.5 mm and, proliferation of cancer cells was observed in the collagen fibers by microscopic observation.

### Example 5

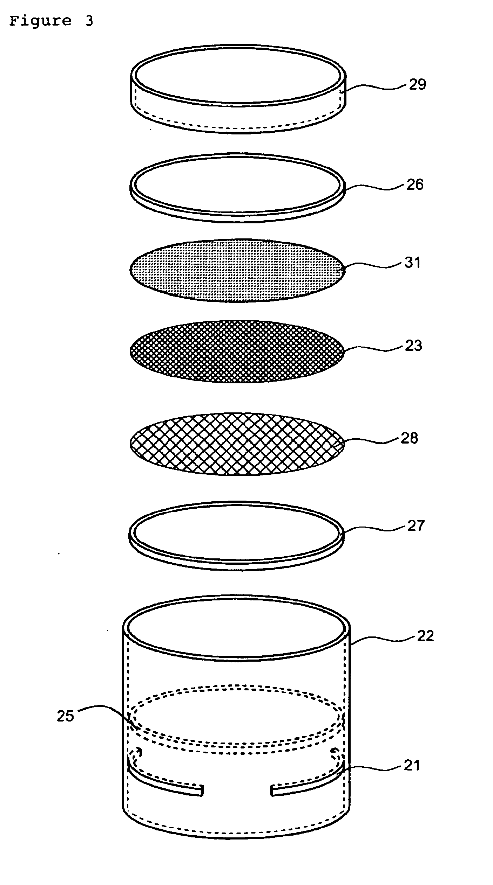

As shown in Fig. 3, the filter paper (24) of the planar gel preparation apparatus used in Example 4 was removed and a sheet (31) having a diameter of 18.7 mm composed of a knit mesh structure of polylactate fibers on the finer metal mesh (23).
250ml of a culture liquid a culture liquid adjusted to contain 0.5 mg/ml of Type I collagen was prepared and placed in this reactor and 2×10⁷ cell/250ml of human fibroblast cells (HFO S2) were circulated for four hours at a flow rate of 5 ml/min.

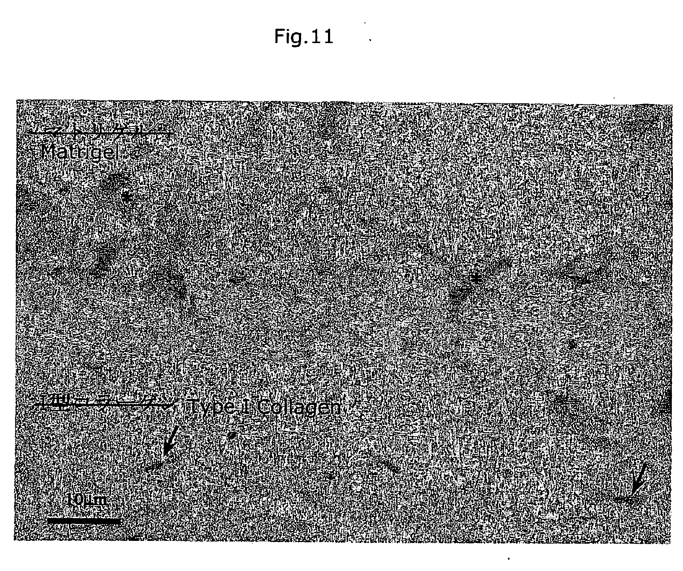

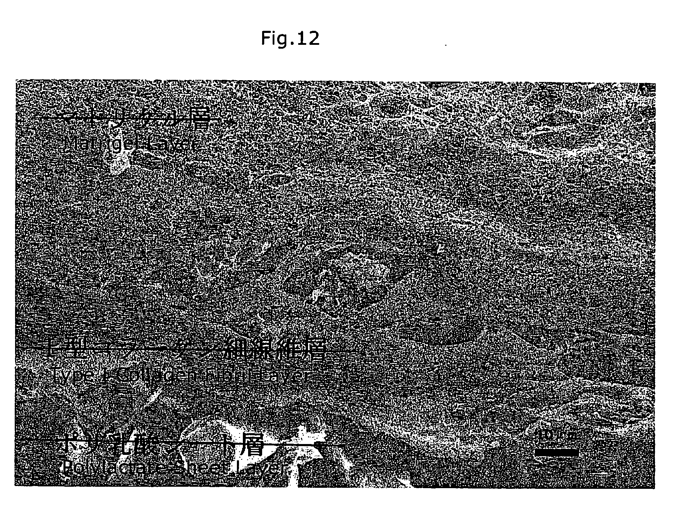

The mixture of 0.5 mg/ml of Type I collagen and fibroblast cells was replaced with 0.1 mg/ml of Matrigel (substitute preparation for basement membrane) and circulation was continued for two hours. Immediately before the completion of the circulation, 4×10⁶ cell of aortic smooth muscle cells (AoSMC; product of Clontech Corp.) were injected into the reactor and the circulation was terminated.
A gel (2.4 mm in thickness, 17.5 mm in diameter) was obtained from inside the reactor. No fetal bovine serum was used in the circulation liquid in this Example.
According to the observation with an optical microscope, a Type I collagen fibril layer containing fibroblast cells and a polymerized Matrigel layer containing smooth muscle cells were formed as layers (Fig. 11).
According to the observation with a scanning electron microscope, a polylactate gel layer, a layer composed of Type I collagen fibrils and fibroblast cells and a layer composed of Matrigel and smooth muscle cells were discriminated (Fig. 12).

### INDUSTRIAL APPLICABILITY

According to the present invention, respectively different layers composed of extracellular matrix components and cells can be stacked by repeating three-dimensional culture and high-density culture. Collagen has a limitation of around 2 mg/ml in a neutral culture liquid at which concentration fibril formation drastically occurs, and therefore collagen gel is difficult to prepare. On the contrary, the closed circulation type high-density culture apparatus of the invention uses a Type I collagen solution having a concentration as low as 0.5 mg/ml, and therefore even a person untrained in cell culturing can prepare a collagen gel with incorporated cells easily.
By utilizing present invention, possibilities of (1) evading the risk of prion contamination which is the pathogen of BSE and (2) regenerating tissues having layered structure such as blood vessel, bowel and ureter.

### [List of reference numerals]

10 Reactor
11 Main body of vessel
12 Lid
13 Holes
14 Center pipe
15 Opening
16 Conduit lines
17 Holes
18 Opening
19 Mesh member
20 Liquid flow controlling member
21 Opening
22 Cylindrical member
23 Planar mesh member
24 Liquid flow controlling member
25 Rib
26, 27 Silicone rings
28 Supporting mesh
29 Inner cylinder
30 Culture medium tank
40 Circulating pump
50 Flow cell
60 Incubator
70 Sensor
80 Display unit
90 Stirrer

## Claims

1. A method of producing a high-density cultured tissue **characterized in that** the method comprises disposing a mesh member and a liquid flow controlling member within a path in which a cell culture liquid containing extracellular matrix components and one or more kinds of animal cells is subjected to circulation culturing so that the liquid flow controlling member is disposed in contact with or close to the mesh member on the back side thereof with regard to the direction of the liquid flow, thereby accumulating the polymerised extracellular matrices and the animal cells highly densely on the surface of the mesh member.

2. The method of producing a high-density cultured tissue according to claim 1 wherein the extracellular matrix component contains one of collagen, elastin, proteoglycan, fibrillin, fibronectin, laminin, chitin, chitosan and chemically modified compounds thereof or a mixture of two or more of these compounds.

3. The method of producing a high-density cultured tissue according to claim 1 wherein the animal cell is a somatic cell, a tumor cell and an embryonic stem cells or a mixture of two or more of these cells.

4. The method of producing a high-density cultured tissue according to claim 3 wherein the somatic cell is selected from a fibroblast, a liver cell, a blood vessel endothelial cell, an epidermic cell, a chondrocyte, a neuroglia, muscle cells and any kind of cells in connective tissue.

5. The method of producing a high-density cultured tissue according to claim 1 wherein the culture liquid further comprises a biologically active substance.

6. The method of producing a high-density cultured tissue according to claim 5 wherein the biologically active substance is one of cell growth factors, hormones and/or natural or synthetic chemical substances having a pharmacological effect or a mixture containing two or more of them.

7. The method of producing a high-density cultured tissue according to claim 1 wherein the culture temperature is 35 to 40°C and the culture time is 6 hours to 9 days.

8. The method of producing a high-density cultured tissue according to claim 1 wherein the liquid flow controlling member is a porous material through which the liquid flow can penetrate.

9. The method of producing a high-density cultured tissue according to claim 7 wherein the porous material through which the liquid flow can penetrate is a filter-paper, a nonwoven fabric or a silk fibroin film.

10. The method of producing a high-density cultured tissue according to claim 1 wherein the mesh member is made of a metal, a ceramic, a synthetic resin or a natural material.

11. The method of producing a high-density cultured tissue according to claim 1 wherein the liquid flow controlling member is incorporated with the mesh member.

12. The method of producing a high-density cultured tissue according to claim 11 wherein the liquid flow controlling member incorporated with the mesh member is an artificial blood vessel.

13. The method of producing a high-density cultured tissue according to claim 1 wherein the mesh member and the liquid flow controlling member are cylindrical and the mesh member is disposed inside of the liquid flow controlling member in the liquid flow from the cylindrical center toward the outer circumference.

14. The method of producing a high-density cultured tissue according to claim 13 wherein the mesh member and the liquid flow controlling member are cylindrical and coaxially disposed, the culture liquid is run from the central axis thereof toward the outer circumference and the culture liquid collected at the outer circumference is circulated to the central axis, thereby accumulating the polymerized extracellular matrix molecules and the animal cells highly densely on the surface of the mesh member.

15. The method of producing a high-density cultured tissue according to claim 1 wherein the mesh member and the liquid flow controlling member are planar and parallelly disposed and the liquid flow controlling member is disposed in contact with or close to the mesh member on the back side thereof with regard to the direction of the liquid flow.

16. The method of producing a high-density cultured tissue according to claim 15 wherein the mesh member and the liquid flow controlling member are horizontally disposed with one above the other in a downward liquid flow.

17. A method of producing a high-density cultured tissue comprising producing a high-density cultured tissue by a method described in any of claims 1 to 16, taking out the obtained high-density cultured tissue and continuing culturing in a non-circulated culture liquid which contains an extracellular matrix component and one or more kinds of animal cells in the same or different formulation.

18. A method of producing a high-density cultured tissue comprising producing a high-density cultured tissue by a method described in any of above 1 to 16, and after taking out the obtained high-density cultured tissue or consecutively, performing at least one operation of forming a different high-density cultured tissue on the above tissue using the same or different culture liquid which contains an extracellular matrix component and one or more kinds of animal cells, thereby forming a laminate type high-density cultured tissue.

19. A method of producing a laminate type high-density cultured tissue comprising taking out a laminate type high-density cultured tissue produced by a method described in claim 18 and continuing culturing in a non-circulated culture liquid which contains an extracellular matrix component and one or more kinds of animal cells in the same or different formulation.

20. The method of producing a high-density cultured tissue according to any of claims 1 to 19 wherein the high-density cultured tissue is skin, cartilage, blood vessel, nerve, muscle or various internal organs.

21. An apparatus for high-density cell culturing which comprises a liquid tank to accommodate a cell culture liquid containing an extracellular matrix component and one or more kinds of animal cells; pump means to circulate the cell culture liquid; a reactor having a cylindrical vessel as a main body and conduit lines which link these elements to constitute a closed circuit, wherein the reactor coaxially keeps a cylindrical mesh member and a cylindrical liquid flow controlling member inside thereof so that the mesh member may be positioned inside the liquid flow controlling member and they may be in contact with or close to each other, and the reactor has an inlet and an outlet of the liquid for circulating the cell culture liquid through the mesh member from the inside to the outside to accumulate the polymerized extracellular matrix molecules and the animal cells on the mesh member.

22. An apparatus for high-density cell culturing which comprises a liquid tank to accommodate a cell culture liquid containing an extracellular matrix component and one or more kinds of animal cells; pump means to circulate the cell culture liquid; a reactor having a cylindrical vessel as a main body and conduit lines which link these elements to constitute a closed circuit, wherein the reactor horizontally keeps a planar mesh member and a planar liquid flow controlling member inside thereof with one above the other so that the mesh member and the liquid flow controlling member may be in contact with or close to each other, and the reactor has an inlet and an outlet of the liquid for circulating the cell culture liquid through the mesh member from the above to the bottom to accumulate the extracellular matrix molecules and the animal cells on the mesh member.

## Patentansprüche

1. Verfahren zur Herstellung eines hochdichten gezüchteten Gewebes, **dadurch gekennzeichnet, dass** das Verfahren das Anordnen eines Maschenteils und eines Flüssigkeitsströmung regulierenden Teils innerhalb eines Wegs, in dem eine Zellzüchtungsflüssigkeit, die extrazelluläre Matrixkomponenten und eine oder mehrere Arten tierischer Zellen enthält, einer Zirkulationszüchtung unterworfen wird, so dass das Flüssigkeitsströmung regulierende Teil in Kontakt mit dem oder in die Nähe des Maschenteils auf der Rückseite desselben mit Bezug auf die Richtung der Flüssigkeitsströmung angeordnet wird, wodurch die polymerisierten extrazellulären Matrizen und die tierischen Zellen hochdicht auf der Oberfläche des Maschenteils angesammelt werden.

2. Verfahren zur Herstellung eines hochdichten gezüchteten Gewebes nach Anspruch 1, wobei die extrazelluläre Matrixkomponente eines von Kollagen, Elastin, Proteoglycan, Fibrillin, Fibronectin, Laminin, Chitin, Chitosan und chemisch modifizierten Verbindungen davon oder eine Mischung von zwei oder mehreren dieser Verbindungen enthält.

3. Verfahren zur Herstellung eines hochdichten gezüchteten Gewebes nach Anspruch 1, wobei die tierische Zelle eine somatische Zelle, eine Tumorzelle und eine Embryostammzelle oder eine Mischung von zwei oder mehreren dieser Zellen ist.

4. Verfahren zur Herstellung eines hochdichten gezüchteten Gewebes nach Anspruch 3, wobei die somatische Zelle unter einem Fibroblast, einer Leberzelle, einer Blutgefäßendothelzelle, einer epidermalen Zelle, einem Chondrozyt, einer Neuroglia, Muskelzellen und irgendeiner Art von Zellen in Bindegewebe ausgewählt ist.

5. Verfahren zur Herstellung eines hochdichten gezüchteten Gewebes nach Anspruch 1, wobei die Züchtungsflüssigkeit des Weiteren eine biologisch aktive Substanz umfasst.

6. Verfahren zur Herstellung eines hochdichten gezüchteten Gewebes nach Anspruch 5, wobei die biologisch aktive Substanz eines ist von Zellwachstumsfaktoren, Hormonen und/oder natürlichen oder synthetischen chemischen Substanzen, die eine pharmakologische Wirkung besitzen, oder eine Mischung, die eine oder zwei davon enthält.

7. Verfahren zur Herstellung eines hochdichten gezüchteten Gewebes nach Anspruch 1, wobei die Züchtungstemperatur 35 bis 40 °C und die Züchtungszeit 6 Stunden bis 9 Tage beträgt.

8. Verfahren zur Herstellung eines hochdichten gezüchteten Gewebes nach Anspruch 1, wobei das Flüssigkeitsströmung regulierende Teil ein poröses Material ist, durch das die Flüssigkeitsströmung hindurchdringen kann.

9. Verfahren zur Herstellung eines hochdichten gezüchteten Gewebes nach Anspruch 7, wobei das poröse Material, durch das die Flüssigkeitsströmung hindurchdringen kann, ein Filterpapier, ein Vliesstoff oder ein Seidefibroinfilm ist.

10. Verfahren zur Herstellung eines hochdichten gezüchteten Gewebes nach Anspruch 1, wobei das Maschenteil aus einem Metall, Keramik, synthetischen Harz oder einem natürlichen Material hergestellt ist.

11. Verfahren zur Herstellung eines hochdichten gezüchteten Gewebes nach Anspruch 1, wobei das Flüssigkeitsströmung regulierende Teil in das Maschenteil eingearbeitet ist.

12. Verfahren zur Herstellung eines hochdichten gezüchteten Gewebes nach Anspruch 11, wobei das Flüssigkeitsströmung regulierende Teil, das in das Maschenteil eingearbeitet ist, ein künstliches Blutgefäß ist.

13. Verfahren zur Herstellung eines hochdichten gezüchteten Gewebes nach Anspruch 1, wobei das Maschenteil und das Flüssigkeitsströmung regulierende Teil zylindrisch sind und das Maschenteil innerhalb des Flüssigkeitsströmung regulierenden Teils in der Flüssigkeitsströmung vom zylindrischen Mittelpunkt auf den äußeren Umfang zu angeordnet ist.

14. Verfahren zur Herstellung eines hochdichten gezüchteten Gewebes nach Anspruch 13, wobei das Maschenteil und das Flüssigkeitsströmung regulierende Teil zylindrisch und koaxial angeordnet sind, die Züchtungsflüssigkeit von der mittigen Achse davon auf den äußeren Umfang zu geführt wird und die Züchtungsflüssigkeit, die an dem äußeren Umfang aufgefangen wird, zu der mittigen Achse zirkuliert wird, wodurch die polymerisierten extrazellulären Matrixmoleküle und die tierischen Zellen hochdicht auf der Oberfläche des Maschenteils angesammelt werden.

15. Verfahren zur Herstellung eines hochdichten gezüchteten Gewebes nach Anspruch 1, wobei das Maschenteil und das Flüssigkeitsströmung regulierende Teil planar und parallel angeordnet sind und das Flüssigkeitsströmung regulierende Teil in Kontakt mit dem oder in der Nähe des Maschenteils auf der Rückseite desselben mit Bezug auf die Richtung der Flüssigkeitsströmung angeordnet ist.

16. Verfahren zur Herstellung eines hochdichten gezüchteten Gewebes nach Anspruch 15, wobei das Maschenteil und das Flüssigkeitsströmung regulierende Teil horizontal eines über dem anderen in einer nach unten gerichteten Flüssigkeitsströmung angeordnet sind.

17. Verfahren zur Herstellung eines hochdichten gezüchteten Gewebes umfassend das Herstellen eines hochdichten gezüchteten Gewebes durch ein Verfahren, das in einem der Ansprüche 1 bis 16 beschrieben ist, das Herausnehmen des erhaltenen hochdichten gezüchteten Gewebes und das Fortsetzen des Züchtens in einer nichtzirkulierten Züchtungsflüssigkeit, die eine extrazelluläre Matrixkomponente und eine oder mehrere Arten tierischer Zellen in derselben oder einer anderen Formulierung enthält.

18. Verfahren zur Herstellung eines hochdichten gezüchteten Gewebes umfassend das Herstellen eines hochdichten gezüchteten Gewebes durch ein Verfahren, das in einem der obigen Ansprüche 1 bis 16 beschrieben ist, und nach dem Herausnehmen des erhaltenen hochdichten gezüchteten Gewebes oder hintereinander das Durchführen mindestens eines Arbeitsgangs des Bildens eines anderen hochdichten gezüchteten Gewebes auf dem obigen Gewebe unter Anwendung derselben oder einer anderen Züchtungsflüssigkeit, die eine extrazelluläre Matrixkomponente und eine oder mehrere Arten tierischer Zellen enthält, wodurch ein hochdichtes gezüchtetes Gewebe vom Laminattyp gebildet wird.

19. Verfahren zur Herstellung eines hochdichten gezüchteten Gewebes von Laminattyp umfassend das Herausnehmen eines hochdichten gezüchteten Gewebes von Laminattyp, das durch ein in Anspruch 18 beschriebenes Verfahren hergestellt worden ist, und das Fortsetzen des Züchtens in einer nichtzirkulierten Züchtungsflüssigkeit, die eine extrazelluläre Matrixkomponente und eine oder mehrere Arten tierischer Zellen in derselben oder einer anderen Formulierung enthält.

20. Verfahren zur Herstellung eines hochdichten gezüchteten Gewebes nach einem der Ansprüche 1 bis 19, wobei das hochdichte gezüchteten Gewebe Haut, Knorpelgewebe, Blutgefäß, Nerv, Muskel oder verschiedene interne Organe ist.

21. Apparat zum Züchten hochdichter Zellen, der Folgendes umfasst: einen Flüssigkeitstank zum Unterbringen einer Zellzüchtungsflüssigkeit, die eine extrazelluläre Matrixkomponente und eine oder mehrere Arten tierischer Zellen enthält; Pumpmöglichkeiten zum Zirkulieren der Zellzüchtungsflüssigkeit; einen Reaktor, der ein zylindrisches Gefäß als Hauptkörper und Führungsleitungen aufweist, die diese Elemente zur Bildung eines geschlossenen Strömungskreises verbinden, wobei der Reaktor koaxial ein zylindrisches Maschenteil und ein zylindrisches Flüssigkeitsströmung regulierendes Teil innerhalb desselben hält, so dass das Maschenteil innerhalb des Flüssigkeitsströmung regulierenden Teils positioniert werden kann und sie sich in Kontakt mit oder nahe aneinander befinden können und der Reaktor einen Einlass und einen Auslass der Flüssigkeit zum Zirkulieren der Zellzüchtungsflüssigkeit durch das Maschenteil von der Innenseite zur Außenseite aufweist, um die polymerisierten extrazellulären Matrixmoleküle und die tierischen Zellen am Maschenteil anzusammeln.

22. Apparat zum Züchten hochdichter Zellen, der Folgendes umfasst: einen Flüssigkeitstank zum Unterbringen einer Zellzüchtungsflüssigkeit, die eine extrazelluläre Matrixkomponente und eine oder mehrere Arten tierischer Zellen enthält; Pumpmöglichkeiten zum Zirkulieren der Zellzüchtungsflüssigkeit; einen Reaktor, der ein zylindrische Gefäß als Hauptkörper und Führungsleitungen aufweist, die diese Elemente zur Bildung eines geschlossenen Strömungskreises verbinden, wobei der Reaktor horizontal ein planares Maschenteil und ein planares Flüssigkeitsströmung regulierendes Teil innerhalb desselben hält, wobei eines über dem anderen liegt, so dass das Maschenteil und das Flüssigkeitsströmung regulierende Teil sich in Kontakt mit oder nahe aneinander befinden können und der Reaktor einen Einlass und einen Auslass der Flüssigkeit zum Zirkulieren der Zellzüchtungsflüssigkeit durch das Maschenteil von oben nach unten aufweist, um die extrazellulären Matrixmoleküle und die tierischen Zellen am Maschenteil anzusammeln.

## Revendications

1. Procédé de production d'un tissu cultivé à densité élevée **caractérisé en ce que** ledit procédé consiste à disposer un élément à mailles et un élément contrôlant l'écoulement de liquide sur un trajet dans lequel un liquide de culture cellulaire contenant des composants de matrice extracellulaire et un ou plusieurs types de cellules animales est soumis à une culture sous circulation de sorte que l'élément contrôlant l'écoulement de liquide est disposé au contact ou à proximité de l'élément à mailles sur la face arrière de ce dernier par rapport à la direction d'écoulement du liquide, ce qui permet d'accumuler les matrices extracellulaires polymérisées et les cellules animales avec une densité élevée sur la surface de l'élément à mailles.

2. Procédé de production d'un tissu cultivé à densité élevée selon la revendication 1 dans lequel le composant de matrice extracellulaire contient un composé parmi le collagène, l'élastine, un protéoglycane, la fibrilline, la fibronectine, la laminine, la chitine, le chitosane et les composés de ceux-ci chimiquement modifiés ou un mélange d'au moins deux de ces composés.

3. Procédé de production d'un tissu cultivé à densité élevée selon la revendication 1 dans lequel la cellule animale est une cellule somatique, une cellule tumorale et une cellule souche embryonnaire ou un mélange d'au moins deux de ces cellules.

4. Procédé de production d'un tissu cultivé à densité élevée selon la revendication 3 dans lequel la cellule somatique est choisie parmi un fibroblaste, une cellule hépatique, une cellule endothéliale de vaisseau sanguin, une cellule épidermique, un chondrocyte, une névroglie, des cellules musculaires et tout type de cellules dans du tissu conjonctif.

5. Procédé de production d'un tissu cultivé à densité élevée selon la revendication 1 dans lequel le liquide de culture comprend en outre une substance biologiquement active.

6. Procédé de production d'un tissu cultivé à densité élevée selon la revendication 5 dans lequel la substance biologiquement active est une substance parmi des facteurs de croissance cellulaire, des hormones et/ou des substances chimiques naturelles ou synthétiques ayant un effet pharmacologique ou un mélange contenant au moins d'eux d'entre elles.

7. Procédé de production d'un tissu cultivé à densité élevée selon la revendication 1 dans lequel la température de culture est de 35 à 40°C et le temps de culture est de 6 heures à 9 jours.

8. Procédé de production d'un tissu cultivé à densité élevée selon la revendication 1 dans lequel l'élément contrôlant l'écoulement de liquide est un matériau poreux à travers lequel l'écoulement de liquide peut pénétrer.

9. Procédé de production d'un tissu cultivé à densité élevée selon la revendication 7 dans lequel le matériau poreux à travers lequel l'écoulement de liquide peut pénétrer est un papier-filtre, un textile non tissé ou un film de fibroïne de soie.

10. Procédé de production d'un tissu cultivé à densité élevée selon la revendication 1 dans lequel l'élément à mailles est fait de métal, de céramique, de résine synthétique ou d'un matériau naturel.

11. Procédé de production d'un tissu cultivé à densité élevée selon la revendication 1 dans lequel l'élément contrôlant l'écoulement de liquide est intégré à l'élément à mailles.

12. Procédé de production d'un tissu cultivé à densité élevée selon la revendication 11 dans lequel l'élément contrôlant l'écoulement de liquide intégré à l'élément à mailles est un vaisseau sanguin artificiel.

13. Procédé de production d'un tissu cultivé à densité élevée selon la revendication 1 dans lequel l'élément à mailles et l'élément contrôlant l'écoulement de liquide sont cylindriques et l'élément à mailles est disposé à l'intérieur de l'élément contrôlant l'écoulement de liquide dans l'écoulement du liquide depuis le centre du cylindre vers la circonférence extérieure.

14. Procédé de production d'un tissu cultivé à densité élevée selon la revendication 13 dans lequel l'élément à mailles et l'élément contrôlant l'écoulement de liquide sont cylindriques et disposés de manière coaxiale, on fait passer le liquide de culture depuis leur axe central vers la circonférence extérieure et on fait circuler le liquide de culture collecté au niveau de la circonférence extérieure en direction de l'axe central, ce qui permet d'accumuler les molécules de matrice extracellulaire polymérisées et les cellules animales avec une densité élevée sur la surface de l'élément à mailles.

15. Procédé de production d'un tissu cultivé à densité élevée selon la revendication 1 dans lequel l'élément à mailles et l'élément contrôlant l'écoulement de liquide sont planaires et disposés parallèlement, et l'élément contrôlant l'écoulement de liquide est disposé au contact ou à proximité de l'élément à mailles sur la face arrière de ce dernier par rapport à la direction d'écoulement du liquide.

16. Procédé de production d'un tissu cultivé à densité élevée selon la revendication 15 dans lequel l'élément à mailles et l'élément contrôlant l'écoulement de liquide sont disposés horizontalement l'un au dessus de l'autre dans un écoulement de liquide vers le bas.

17. Procédé de production d'un tissu cultivé à densité élevée consistant à produire un tissu cultivé à densité élevée par un procédé décrit dans l'une quelconque des revendications 1 à 16, à sortir le tissu cultivé à densité élevée ainsi obtenu et à continuer la culture dans un liquide de culture non circulant qui contient un composant de matrice extracellulaire et un ou plusieurs types de cellules animales dans la même formulation ou une formulation différente.

18. Procédé de production d'un tissu cultivé à densité élevée consistant à produire un tissu cultivé à densité élevée par un procédé décrit dans l'une quelconque des revendications 1 à 16, et après avoir sorti le tissu cultivé à densité élevée ainsi obtenu ou simultanément, à exécuter au moins une opération de formation d'un tissu cultivé à densité élevée différent sur le tissu ci-dessus à l'aide du même liquide de culture ou d'un liquide de culture différent qui contient un composant de matrice extracellulaire et un ou plusieurs types de cellules animales, ce qui permet de former un tissu cultivé à densité élevée de type stratifié.

19. Procédé de production d'un tissu cultivé à densité élevée de type stratifié consistant à sortir un tissu cultivé à densité élevée de type stratifié produit par un procédé décrit dans la revendication 18 et à continuer la culture dans un liquide de culture non circulant qui contient un composant de matrice extracellulaire et un ou plusieurs types de cellules animales dans la même formulation ou une formulation différente.

20. Procédé de production d'un tissu cultivé à densité élevée selon l'une quelconque des revendications 1 à 19 dans lequel le tissu cultivé à densité élevée est de la peau, du cartilage, un vaisseau sanguin, un nerf, un muscle ou divers organes internes.

21. Appareil permettant la culture de cellules à haute densité qui comprend un réservoir à liquide destiné à recevoir un liquide de culture cellulaire contenant un composant de matrice extracellulaire et un ou plusieurs types de cellules animales; des moyens de pompage pour faire circuler le liquide de culture cellulaire; un réacteur ayant une cuve cylindrique en tant que corps principal et des conduites qui relient ces éléments pour constituer un circuit fermé, dans lequel le réacteur maintient coaxialement un élément à mailles cylindrique et un élément contrôlant l'écoulement de liquide cylindrique à l'intérieur du réacteur de façon que l'élément à mailles puisse être positionné à l'intérieur de l'élément contrôlant l'écoulement de liquide et qu'ils puissent être en contact l'un avec l'autre ou à proximité l'un de l'autre, et le réacteur comporte une entrée et une sortie pour le liquide permettant de faire circuler le liquide de culture cellulaire à travers l'élément à mailles de l'intérieur vers l'extérieur pour accumuler les molécules de matrice extracellulaire polymérisées et les cellules animales sur l'élément à mailles.

22. Appareil permettant la culture de cellules à haute densité qui comprend un réservoir à liquide destiné à recevoir un liquide de culture cellulaire contenant un composant de matrice extracellulaire et un ou plusieurs types de cellules animales; des moyens de pompage pour faire circuler le liquide de culture cellulaire; un réacteur ayant une cuve cylindrique en tant que corps principal et des conduites qui relient ces éléments pour constituer un circuit fermé, dans lequel le réacteur maintient horizontalement un élément à mailles planaire et un élément contrôlant l'écoulement de liquide planaire à l'intérieur du réacteur, l'un étant au-dessus de l'autre, de façon que l'élément à mailles et l'élément contrôlant l'écoulement de liquide puissent être en contact l'un avec l'autre ou à proximité l'un de l'autre, et le réacteur comporte une entrée et une sortie pour le liquide permettant de faire circuler le liquide de culture cellulaire à travers l'élément à mailles de haut en bas pour accumuler les molécules de matrice extracellulaire et les cellules animales sur l'élément à mailles.
